# EUROPEAN PATENT SPECIFICATION

(11) **EP 1 401 386 B1**
(45) Date of publication and mention of the grant of the patent: **30.06.2010**
(21) Application number: 02705551.6
(22) Date of filing: 11.03.2002
(51) Int. Cl.: A61K 8/00

(54) **COSMETIC COMPOSITION CONTAINING THE EXTRACT OF MOUNTAIN GINSENG ADVENTITIOUS ROOTS**
KOSMETISCHE ZUBEREITUNG ENTHALTEND EINEN EXTRAKT VON DEN NEBENWURZELN DES BERGGINSENGS
COMPOSITION COSMETIQUE CONTENANT L'EXTRAIT DE RACINES ADVENTIVES DE GINSENG DES MONTAGNES

(30) Priority: 04.07.2001 KR 2001039685
(43) Date of publication of application: 31.03.2004
(73) Proprietor: Hankook Cosmetics Co., Ltd., Seoul 110-790 (KR)
(72) Inventor: JOUNG, Min-seok, Seoul 138-906 (KR); SEO, Bong-seok, Wonmi-gu, Buchon, Kyonggi-do 421-150 (KR); CHOI, Jong-oan, Wonmi-gu, Buchon, Kyonggi-do 420-719 (KR); JOO, Yong-ho, Yangchon-gu, Seoul 158-769 (KR)
(74) Representative: Gaunt, Robert John
(86) International application number: PCT/KR2002/000415
(87) International publication number: WO 2003/003996

(56) References cited:
- JP-A- 4 009 316
- JP-A- 8 092 055
- JP-A- 8 092 056
- KR-A- 2000 062 017
- US-A- 5 571 503
- US-B- 6 190 664

## Description

### TECHNICAL FIELD

The present invention relates, generally, to a cosmetic composition and, in particular, to a cosmetic composition containing an extract from mountain ginseng (also called wild ginseng) adventitious roots which are grown through tissue culture.

### BACKGROUND ART

There are many factors causing the human skin to turn dark. Of them, exposure to UV light is well known. Once the skin is exposed to UV light, melanin is actively synthesized in the skin cell melanocytes and released to the epidermis. The synthesis of melanin in melanocytes is mediated by tyrosinase. In detail, cellular tyrosine is converted by tyrosinase into dopaquinone from which the pigment melanin is produced through spontaneous enzymatic reactions. Thus, the production of melanin can be inhibited by blocking some reactions of the melanin synthesis pathway, and this concept is adopted in typical methods of restraining the darkening of the skin.

In this regard, ascorbic acid, kojic acid, arbutin, hydroquinone, lipid-soluble licorice extracts, and various vegetable extracts are conventionally used. By chelating copper ions present in the active site of the tyrosinase, kojic acid functions to inhibit the activity of the enzyme. Despite its high inhibitory activity against tyrosinase, kojic acid is not used in cosmetic materials owing to inferior compatibility with other cosmetic materials. Hydroquinone is not used in cosmetic materials owing to safety problems including irritation of the skin.

Much research has been and continues to be directed to the finding of natural materials, especially herbs capable of whitening the skin. As a result, extracts from various herbs including Morus alba LINNE, Glycyrrhiza uralensis FISCH Glyrhiza inflata BAT, Paeonia lactiflora PALL. P. veitchii LYNCH, Cinnamomum loureirii NEES, Sophora Flavescens Arr., Pueraria thunbergiana(SIEB.et ZUCC) BENTH, Angelica sinensis(OLIV.)DIELS, Paeonia suffruticosa ANDR., Pinellia ternata(THUNB.)BREIT, aloe, etc., are found to be inhibitory of the activity of tyrosinase. However, these herbs do not show definite whitening effects. Additionally, various experiments demonstrate that the herb extracts cannot be used in cosmetically or medicinally effective amounts in the aspects of stability and discoloration of final products. Particularly, only when being used at high concentrations, the herb extracts are, for the most part, inhibitory of the activity of tyrosinase. That is, most of the herb extracts at low concentrations scarcely show inhibitory activity against tyrosinase.

Therefore, there remains a need for an improved cosmetic material that is excellent in terms of skin whitening effect and compatibility with other cosmetic materials, with no irritation of the skin.

### DISCLOSURE OF THE INVENTION

Leading to the present invention, the intensive and thorough research into natural whitening materials, conducted by the present inventors with an aim to solve the problems encountered in prior arts, resulted in the finding that mountain ginseng extracts strongly inhibit the production of melanin in melanocytes and thus show excellent skin whitening effects.

Thus, it is an object of the present invention to provide a mountain ginseng extract which is effective in whitening the skin and can be used as a cosmetic material.

It is another object of the present invention to provide a skin-whitening cosmetic formulation which contains a mountain ginseng adventitious roots extract as an effective ingredient.

It is a further object of the present invention to provide a method for preparing a skin-whitening cosmetic formulation comprising a mountain ginseng adventitious roots extract as a cosmetically effective ingredient.

In accordance with an aspect of the present invention, there is provided a cosmetic composition, comprising an extract from tissue-cultured mountain ginseng adventitious roots in an amount of 0.1 to 10.0 % by weight.

In accordance with another aspect of the present invention, there is provided a method for preparing an extract from tissue-cultured mountain ginseng adventitious roots, comprising the steps of: drying the tissue-cultured mountain ginseng adventitious roots; immersing the roots in a solution comprising ethanol; filtering the solution; and optionally removing ethanol from the filtrate.

### BRIEF DESCRIPTION OF THE DRAWING

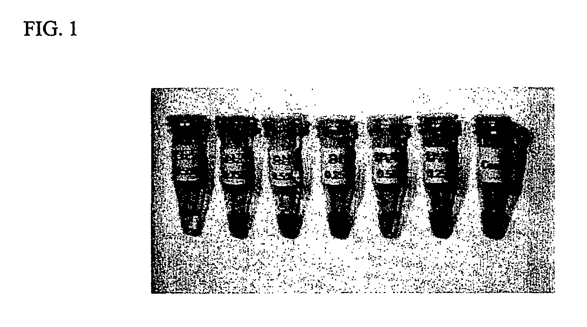

Fig. 1 is a microphotograph showing the inhibition of the synthesis of melanin in B-16 melanoma cells treated with a mountain ginseng adventitious roots extract (0.5% or 0.25%, vial Nos. 1 and 2, respectively), a red ginseng extract (0.5% or 0.25%, vial Nos. 3 and 4, respectively), arbutin (0.5 or 0.25%, vial Nos. 5 and 6, respectively,) and control (vial No. 7).

### BEST MODES FOR CARRYING OUT THE INVENTION

In plant taxonomy, mountain ginseng, whose origin is estimated to date back to about one hundred million years ago, is classified as a phanerogam and an angiosperm belonging to Araliacae family. In Korea, the first mountain ginseng was reported to be found at Mt. Mohoo. Mountain ginseng requires very delicate environmental conditions for its growth. For instance, when placed in an environment unsuitable for their germination, seeds of mountain ginseng remain buried for a long period. That is, they wait until they meet suitable environmental conditions.

Mountain ginseng is classified into 18 species according to the conditions under which they grow. For instance, mountain ginseng is called heavenly species (natural mountain ginseng) if the seed is-spontaneously sown and germinated; earthy species if the seed is eaten by wild animals and excreted therefrom; human species if the seed is sown artificially; cultured mountain ginseng if it is obtained by implanting grown ginseng in a. mountain; and quasi-mountain ginseng if young mountain ginseng is cultured artificially. Among them, natural mountain ginseng has the most potent medicinal effects. With abundant biologically active substances, such as saponin, different from those of other species, the natural mountain ginseng was experimentally proven to have a variety of activities including immunity enhancement, inhibitory activity against cancer, anti-diabetes mellitus activity, cardio-stimulation, reduction of blood pressure and hypotension, stimulation of liver, stomach and brain functions, anti-aging activity, restorative activity, etc. In fact, mountain ginseng is known as a mysterious oriental medicinal herb.

No reports have been disclosed concerning the skin whitening effect of mountain ginseng.

In the present invention, an extract from mountain ginseng adventitious roots is used in cosmetics for whitening the skin. Mountain ginseng adventitious roots are obtained by tissue culture.

In accordance with the present invention, a cosmetic formulation for whitening the skin contains an extract from mountain ginseng adventitious roots as a cosmetically effective ingredient in an amount of 0.1 to 10.0 % by weight and preferably in an amount of 0.5 to 2.0 % by weight.

To extract, from mountain ginseng adventitious roots, materials effective for use in whitening cosmetics, first, tissue-cultured mountain ginseng adventitious roots are dried. The dry mountain ginseng adventitious roots are immersed in a solution containing ethanol, and aged for a predetermined period of time. The solution containing extracts from the mountain ginseng roots are filtered. Optionally, ethanol may be removed from the filtrate.

The solution used for the extraction of mountain ginseng adventitious roots may contain ethanol alone or in combination with other alcohols, such as 1,3-butylene glycol and propylene glycol. When employing ethanol alone, the solution is preferably a 70 % aqueous solution, and used in an amount 20-40 fold larger than the weight of the mountain ginseng adventitious roots used. On the other hand, when ethanol is used in combination with 1,3-butylene glycol or propylene-glycol is used, a 70% ethanol solution is added in an amount 20-40 fold larger than the weight of the mountain ginseng adventitious roots, while 1,3-butylene glycol or propylene glycol is used in an amount 2-10 fold larger than the weight of the mountain ginseng adventitious roots. The mountain ginseng adventitious root extract may be formulated along with at least one species for cosmetics.

Having generally described this invention, a further understanding can be obtained by reference to certain specific examples which are provided herein for purposes of illustration only and are not intended to be limiting unless otherwise specified.

### 1. PREPARATION OF EXTRACT FROM MOUNTAIN GINSENG

### EXAMPLE 1

After being dried, 1 kg of tissue-cultured mountain ginseng adventitious roots was immersed in 30 kg of an aqueous 70 % ethanol solution and aged for a sufficient time. Then, the solution was filtered through a filter paper (5C, 185 mm, Toyoroshi Kaisha, Ltd.) to obtain an extract.

### EXAMPLE 2

After being dried, 1 kg of tissue-cultured mountain ginseng adventitious roots was immersed in 30 kg of an aqueous 70 % ethanol solution and aged for a sufficient time. Then, the solution was filtered through a filter paper (5C, 185 mm, Toyoroshi Kaisha, Ltd.). From the filtrate thus obtained, ethanol was removed by heating at 100°C in a water bath to obtain an extract in a syrup state.

### EXAMPLE 3

After being dried, 1 kg of tissue-cultured mountain ginseng adventitious roots was immersed in 30 kg of an aqueous 70 % ethanol solution and 3 kg of 1,3-butylene glycol or propylene glycol, and aged for a sufficient time. Then, the solution was filtered through a filter paper (5C, 185 mm, Toyoroshi Kaisha, Ltd.), followed by the removal of ethanol from the filtrate at 50-60°C to obtain an extract.

### 2. TEST FOR INHIBITORY ACTIVITY AGAINST PRODUCTION OF MELANIN AND FOR CYTOTOXICITY

### EXPERIMENTAL EXAMPLE 1: Inhibitory Activity Against Production of Melanin

Extracts prepared from tissue-cultured mountain ginseng adventitious roots in Examples 1 to 3 were examined for skin-whitening effects using B-16 melanoma cells (obtained from Yonsei University Medical College at Wonju, Korea). In this regard, B-16 melanoma cells, derived from black mice, were added in a dose of 2x10⁴ cells/well in a 6 well tissue culture plate, each well containing 2 ml of a DMEM medium (Gibco, U.S.A.) supplemented with 10% FBS(Gibco, U.S.A.), followed by the incubation at 37°C for 24 hours in a 5% CO₂ atmosphere. Thereafter, the culture medium was replaced with a 10% FBS-supplemented DMEM medium containing 2 µM α-MSH (melanocyte stimulating hormone, commercially available from Sigma, U.S.A.) and 2 mM theophylline (Sigma, U.S.A.). Afterwards, the extracts were diluted in the same medium and added to the cells which were then cultured at 37°C in a 5% CO₂ atmosphere until cells covered 80% of the bottom area of the well. After the removal of the medium, the cells were washed with PBS (phosphate buffered saline, Gibco, U.S.A.) and treated with trypsin to obtain cell pellets. The collected cell pallets were centrifuged at 5,000 rpm for 10 min and the supernatant was removed. The cell pellets thus obtained were dried at 60°C for 24 hours in an incubator, after which 1 N NaOH was added to the cells to dissolve cellular melanin. For visual examination, the dissolved melanin was photographed by use of a video microscope and the results are given in Fig. 1. Also, dilutions of the melanin were analyzed through ELISA to examine the extracts from mountain ginseng adventitious roots for inhibitory activity against melanin synthesis. The results are given in Table 1, below.

**TABLE 1**

| Inhibitory Activity Against Melanin Synthesis | | | | |
|---|---|---|---|---|
| Sample | Concentration | O.D. at 490 nm | | Inhibition |
| | (µm/mℓ) | Sample (× 3) | Control (×3) | Activity Rate (%) |
| Mt. Ginseng | 2500 | 0.154 | 0.976 | 84 |
| Adventitious Roots Extract | 5000 | 0.052 | 0.982 | 95 |
| Red Ginseng | 2500 | 0.665 | 0.972 | 32 |
| Extract | 5000 | 0.399 | 0.988 | 60 |
| Arbutin | 2500 | 0.218 | 0.980 | 78 |
| | 5000 | 0.102 | 0.986 | 90 |

As seen in Fig. 1 and Table 1, the extracts from mountain ginseng adventitious roots show excellent whitening effects on the skin.

### EXPERIMENTAL EXAMPLE 2: Cytotoxicity

Extracts from tissue-cultured mountain ginseng adventitious roots, prepared in Examples 1 to 3, were examined for cytotoxicity, in comparison with a red ginseng extract and arbutin, which are conventionally used as skin-whitening materials.

After being recovered by trypsinization, the transformed mouse fibroblast L929 cells were suspended in DMEM supplemented with 2% BCS (bovine calf serum), and the suspension was aliquoted at a concentration of 2,500-3,000 cells/well into each well of 96 well tissue culture plates, followed by incubation at 37°C for 48 hours in a 5% CO₂ atmosphere. After substitution of the medium with a fresh, serum-free one, the cells were treated with the test materials shown in Table 2, below, and cultured for 48 hours. To each well, Neutral Red (50 µg/ml) was added, and reacted with cells for 3 hours. After completion of the accumulation of Neutral Red in live cells, the dye was fixed with the use of 1.0% formalin/1.0% CaCl₂. Neutral Red was extracted from cells using 1.0% acetic acid/50% ethanol solution. The extracted Neutral Red was analyzed by ELISA to measure the cytotoxicity of the test materials according to concentrations. The results are given in Table 2, below.

**TABLE 2**

| Cytotoxicity | | | | | | | |
|---|---|---|---|---|---|---|---|
| Sample | Concentration (w/w%) | | | | | | NR₅₀ |
| | 4 | 2 | 1 | 0.1 | 0.01 | 0.001 | |
| Mt. Ginseng Adventitious Roots Extract | 2 | 71 | 83 | 100 | 100 | 100 | 2.47 |
| Red Ginseng Extract | - | 2 | 51 | 87 | 100 | 100 | 1.01 |
| Arbutin | - | - | 2 | 63 | 82 | 98 | 0.29 |

As apparent from its far higher NR₅₀ than that of conventional materials, mountain ginseng adventitious roots extract is very safe to the body.

### 3. FORMULATIONS OF SKIN-WHITENING COSMETICS

Various cosmetics containing the extract from tissue-cultured mountain ginseng adventitious roots were prepared as follows:

### FORMULATION EXAMPLE 1

A skin lotion was prepared according to the formulation shown in Table 3, below.

**TABLE 3**

| Ingredient | Content (Wt%) |
|---|---|
| Mt. Ginseng Adventitious Roots Extract | 2.00 |
| 1,3-Butylene Glycol | 2.00 |
| Glycerin | 3.00 |
| Oleylalcohol | 2.00 |
| Polysorbate 20 | 1.00 |
| Ethanol | 6.00 |
| Preservative | Trace |
| Flavor | Trace |
| Pure Water | Remainder |
| Total | 100.00 |

### FORMULATION EXAMPLE 2

A milk lotion was prepared according to the formulation shown in Table 4, below.

**TABLE 4**

| Ingredient | Content (Wt%) |
|---|---|
| Mt. Ginseng Adventitious Roots Extract | 2.00 |
| 1,3-Butylene Glycol | 4.00 |
| Glycerin | 3.00 |
| Oleylalcohol | 0.50 |
| Polysorbate 20 | 1.00 |
| Preservative | Trace |
| Flavor | Trace |
| Pure Water | Remainder |
| Total | 100.00 |

### FORMULATION EXAMPLE 3

A nutritional cream was prepared according to the formulation shown in Table 5, below.

**TABLE 5**

| Ingredient | Content (Wt%) |
|---|---|
| Mt. Ginseng Adventitious Roots Extract | 2.00 |
| Propylene Glycol | 5.00 |
| Glycerin | 5.00 |
| Triethanol Amine | 0.50 |
| Tocopheryl Acetate | 1.00 |
| Liquid Paraffin | 5.00 |
| Squalane | 5.00 |
| Macadamia nut oil | 15.00 |
| Polysorbate 60 | 1.00 |
| Sorbitan Cesquiolate | 1.00 |
| Carboxylvinyl Polymer | 0.20 |
| Preservative | Trace |
| Flavor | Trace |
| Pure Water | Remainder |
| Total | 100.00 |

### FORMULATION EXAMPLE 4

A massage cream was prepared according to the formulation shown in Table 6, below.

**TABLE 6**

| Ingredient | Content (Wt%) |
|---|---|
| Mt. Ginseng Adventitious Roots Extract | 2.00 |
| Vacerin | 5.00 |
| Liquid Paraffin | 30.00 |
| Beeswax | 3.00 |
| Polysorbate 60 | 1.00 |
| Sorbitan Cesquiolate | 1.00 |
| Propylene Glycol | 5.00 |
| Glycerin | 5.00 |
| Preservative | Trace |
| Flavor | Trace |
| Pure Water | Remainder |
| Total | 100.00 |

### FORMULATION EXAMPLE 5

An essence was prepared according to the formulation shown in Table 7, below.

**TABLE 7**

| Ingredient | Content (Wt%) |
|---|---|
| Mt. Ginseng Adventitious Roots Extract | 2.00 |
| 1.3-Butylene Glycol | 2.00 |
| Glycerin | 5.00 |
| Triethanol Amine | 0.27 |
| Carboxyvinyl Polymer | 0.20 |
| Xanthan Gum | 0.10 |
| Preservative | Trace |
| Flavor | Trace |
| Pure Water | Remainder |
| Total | 100.00 |

### FORMULATION EXAMPLE 6

A gel-phase cream was prepared according to the formulation shown in Table 8, below.

**TABLE 8**

| Ingredient | Content (Wt%) |
|---|---|
| Mt. Ginseng Adventitious Roots Extract | 2.00 |
| Propylene Glycol | 20.00 |
| Carbomer | 20.00 |
| Polysorbate 60 | 0.50 |
| Preservative | Trace |
| Flavor | Trace |
| Pure Water | Remainder |
| Total | 100.00 |

### FORMULATION EXAMPLE 7

A skin pack formulation was prepared with the ingredients shown in Table 9, below.

**TABLE 9**

| Ingredient | Content (Wt%) |
|---|---|
| Mt. Ginseng Adventitious Roots Extract | 2.00 |
| 1.3-Butylene Glycol | 5.00 |
| Polyvinyl Alcohol | 12.00 |
| Polyvinyl Pyrrolidone | 2.00 |
| Ethanol | 8.00 |
| Polyvinyl Acetate | 1.00 |
| Preservative | Trace |
| Flavor | Trace |
| Pure Water | Remainder |
| Total | 100.00 |

### FORMULATION EXAMPLE 8

A nutritional emulsion was prepared according to the formulation shown in Table 10, below.

**TABLE 10**

| Ingredient | Content (Wt%) |
|---|---|
| Mt. Ginseng Adventitious Roots Extract | 2.00 |
| Propylene Glycol | 5.00 |
| Glycerin | 4.00 |
| Squalane | 5.00 |
| Macadamia nut oil | 10.00 |
| Polysorbate 60 | 0.50 |
| Cetearyl Alcohol | 1.00 |
| Carbomer | 0.20 |
| Preservative | Trace |
| Flavor | Trace |
| Pure Water | Remainder |
| Total | 100.00 |

### INDUSTRIAL APPLICABILITY

As described hereinbefore, a cosmetic composition comprising an extract from tissue-cultured mountain ginseng adventitious roots is provided for whitening the skin, in accordance with the present invention. The cosmetic composition containing a mountain ginseng adventitious roots extract as an essential ingredient is excellent in terms of whitening effect, without irritation of the skin.

The present invention has been described in an illustrative manner, and it is to be understood that the terminology used is intended to be in the nature of description rather than of limitation. Many modifications and variations of the present invention are possible in light of the above teachings. Therefore, it is to be understood that within the scope of the appended claims, the invention may be practiced otherwise than as specifically described.

## Claims

1. A cosmetic composition comprising an extract from mountain ginseng adventitious roots in an amount of 0.1 to 10.0% by weight based on the total weight of the composition, said adventitious roots being obtained by tissue culture of naturally-occurring mountain ginseng.

2. The cosmetic composition as set forth in claim 1, wherein the extract is contained in an amount of 0.5 to 2.0 % by weight based on the total weight of the composition.

3. The cosmetic composition as set forth in claim 1, wherein the extract is obtained by a process comprising the steps of: drying said adventitious roots; immersing the roots in a solvent; and removing solids to obtain the extract.

4. The cosmetic composition as set forth in claim 3, wherein the solvent is selected from the group consisting of an 70% aqueous ethanol, mixtures of ethanol and 1,3-butylene glycol, and mixtures of ethanol and propylene glycol.

5. The cosmetic composition as set forth in claim 1, which is in a formulation form selected from the group consisting of a skin lotion, a milk lotion, an emulsion cream, a massage cream, an essence, a gel-phase cream, a pack, and a nutritional emulsion.

6. A method for preparing an extract from tissue-cultured mountain ginseng adventitious roots, comprising the steps of:
drying the tissue-cultured mountain ginseng adventitious roots;
immersing the dried adventitious roots in an ethanol-containing solvent;
separating the solvent from solids to obtain the extract; and
optionally removing ethanol from the extract.

7. The method as set forth in claim 6, wherein the ethanol-containing solution is selected from the group consisting of an 70% aqueous ethanol, mixtures of ethanol and 1,3-butylene glycol, and mixtures of ethanol ad propylene glycol.

8. The method as set forth in claim 6, wherein the separation step is filtration.

## Patentansprüche

1. Kosmetische Zusammensetzung, umfassend einen Extrakt aus Bergginsengadventivwurzeln in einer Menge von 0,1 bis 10,0 Gew.-%, basierend auf dem Gesamtgewicht der Zusammensetzung, wobei die Adventivwurzeln durch Gewebekultur von natürlich vorkommendem Bergginseng erhalten werden.

2. Kosmetische Zusammensetzung, wie in Anspruch 1 dargelegt, wobei der Extrakt in einer Menge von 0,5 bis 2,0 Gew.-%, basierend auf dem Gesamtgewicht der Zusammensetzung, enthalten ist.

3. Kosmetische Zusammensetzung, wie in Anspruch 1 dargelegt, wobei der Extrakt durch ein Verfahren erhalten wird, umfassend die Schritte des Trocknens der Adventivwurzeln, des Eintauchens der Wurzeln in ein Lösungsmittel und des Entfernens von Feststoffen unter Erhalt des Extraktes.

4. Kosmetische Zusammensetzung, wie in Anspruch 3 dargelegt, wobei das Lösungsmittel aus der Gruppe ausgewählt ist, bestehend aus 70%igem wässerigem Ethanol, Gemischen aus Ethanol und 1,3-Butylenglycol und Gemischen aus Ethanol und Propylenglycol.

5. Kosmetische Zusammensetzung, wie in Anspruch 1 dargelegt, welche in einer Formulierungsform vorliegt, ausgewählt aus der Gruppe, bestehend aus einer Hautlotion, einer Milchlotion, einer Emulsionscreme, einer Massagecreme, einer Essenz, einer Gelphasencreme, einer Gesichtspackung und einer Nähremulsion.

6. Verfahren zur Herstellung eines Extraktes aus gewebekultivierten Bergginsengadventivwurzeln, umfassend die Schritte:
Trocknen der gewebekultivierten Bargginsengadventivwurzeln;
Eintauchen der getrockneten Adventivwurzeln in ein Ethanol enthaltendes Lösungsmittel;
Trennen des Lösungsmittels von den Feststoffen unter Erhalt des Extraktes und
gegebenenfalls Entfernen des Ethanols aus dem Extrakt.

7. Verfahren, wie in Anspruch 6 dargelegt, wobei die Ethanol enthaltende Lösung aus der Gruppe ausgewählt ist, bestehend aus 70%igem wässerigem Ethanol, Gemischen aus Ethanol und 1,3-Butylenglycol und Gemischen aus Ethanol und Propylenglycol.

8. Verfahren, wie in Anspruch 6 dargelegt, wobei der Trennungsschritt Filtration ist.

## Revendications

1. Composition cosmétique comprenant un extrait de racines adventives de ginseng des montagnes en une quantité de 0,1 à 10,0 % en poids sur la base du poids total de la composition, lesdites racines adventives étant obtenues par culture de tissu de ginseng des montagnes naturel.

2. Composition cosmétique selon la revendication 1 où l'extrait est contenu en une quantité de 0,5 à 2,0 % en poids sur la base du poids total de la composition.

3. Composition cosmétique selon la revendication 1 où l'extrait est obtenu par un procédé comprenant les étapes de : séchage desdites racines adventives ; immersion des racines dans un solvant ; et retrait des solides pour obtenir l'extrait.

4. Composition cosmétique selon la revendication 3 où le solvant est choisi dans le groupe consistant en un éthanol aqueux à 70 %, les mélanges d'éthanol et de 1,3-butylèneglycol, et les mélanges d'éthanol et de propylèneglycol.

5. Composition cosmétique selon la revendication 1 qui est dans une forme de formulation choisie dans le groupe consistant en une lotion pour la peau, une lotion de type lait, une crème en émulsion, une crème pour le massage, une essence, une crème à phase de gel, un pack et une émulsion nutritionnelle.

6. Procédé pour préparer un extrait de racines adventives de ginseng des montagnes cultivées en culture de tissu comprenant les étapes de :
séchage des racines adventives de ginseng des montagnes cultivées en culture de tissu ;
immersion des racines adventives séchées dans un solvant contenant de l'éthanol ;
séparation du solvant des solides pour obtenir l'extrait ; et
éventuellement retrait de l'éthanol de l'extrait.

7. Procédé selon la revendication 6 où la solution contenant de l'éthanol est choisie dans le groupe consistant en un éthanol aqueux à 70 %, les mélanges d'éthanol et de 1,3-butylèneglycol et les mélanges d'éthanol et de propylèneglycol.

8. Procédé selon la revendication 6 où l'étape de séparation est une filtration.
